# EUROPÄISCHE PATENTSCHRIFT

(11) **EP 0 559 676 B1**
(45) Veröffentlichungstag und Bekanntmachung des Hinweises auf die Patenterteilung: **10.04.1996**
(21) Anmeldenummer: 91920010.5
(22) Anmeldetag: 18.11.1991
(51) Int. Cl.: C12N 9/00, C07K 1/36, C12N 1/02, C12N 9/50, C12N 9/54, C12N 9/14

(54) **FÄLLUNGSVERFAHREN FÜR EXOCELLULÄRE PROTEINE**
PRECIPITATION PROCESS FOR EXOCELLULAR PROTEINS
PROCEDE DE PRECIPITATION POUR PROTEINES EXOCELLULAIRES

(30) Priorität: 26.11.1990 DE 4037530
(43) Veröffentlichungstag der Anmeldung: 15.09.1993
(73) Patentinhaber: Henkel Kommanditgesellschaft auf Aktien, D-40191 Düsseldorf (DE)
(72) Erfinder: WEISS, Albrecht, D-4018 Langenfeld (DE); BERKE, Wolfgang, D-5067 Kürten-Dürscheid (DE)
(86) Internationale Anmeldenummer: EP9102170
(87) Internationale Veröffentlichungsnummer: WO9209687

(56) Entgegenhaltungen:
- EP-A- 0 216 270
- EP-A- 0 325 348
- WO-A-90/13632
- DE-A- 1 492 302
- DE-A- 1 810 823
- US-A- 3 101 302
- US-A- 3 711 462
- CHEMICAL ABSTRACTS, Band 107, Nr. 15, 12 Oktober 1987, Columbus, OH (US); Seite 345, Nr. 129968d
- CHEMICAL ABSTRACTS, Band 87, Nr. 1, 04 Juli 1976, Columbus, OH (US); I. ALEZADEH et al., Seite 334, Nr. 4003x

## Beschreibung

Die Erfindung betrifft ein Verfahren zur Reinigung und Abtrennung von exocellulären Proteinen aus Fermenterbrühen. Insbesondere betrifft die Erfindung ein Verfahren zur Abtrennung der Enzyme in fester Form, wobei Farb- und Geruchsstoffe entfernt werden.

Zahlreiche Enzyme, insbesondere Hydrolasen wie beispielsweise Proteasen, Amylasen oder Lipasen, werden durch Fermentation von Mikroorganismen hergestellt. Geeignete Mikroorganismen und Herstellverfahren sind beispielsweise in den folgenden Patenten bzw. Patentanmeldungen beschrieben: DE 18 00 508, DE 22 24 777, DE 25 51 742, US 3 827 938, WO 88/01293, DE 18 07 185, US 3 740 318, DE 23 34 463, DE 20 26 092, EP 0 232 169, EP 0 220 921, EP 0 247 647 und EP 0 246 678.

Für zahlreiche Anwendungen, z.B. für den Einsatz der Enzymlösungen in Flüssigwaschmitteln sind stark färbende oder riechende Beimengungen nicht tolerierbar. Bei der technischen Herstellung der Enzyme versucht man daher die Verunreinigungen durch Fällungsverfahren abzutrennen. Bisher bekannt gewordene Fällungsverfahren haben jedoch den Nachteil, daß zur Erzielung einer guten Farbqualität erhebliche Ausbeuteverluste in Kauf genommen werden müssen.

Um diesen Schwierigkeiten zu begegnen, wird in der deutschen Patentanmeldung P 39 11 099.0 ein Fällungsverfahren beschrieben, bei dem man eine durch Fermentation hergestellte Enzymlösung mit einem Maskierungsmittel versetzt und hernach eine Fällung erzeugt, indem man in beliebiger Reihenfolge zwei wasserlösliche, sich gegenseitig fällende, ionische Verbindungen zugibt, und gewünschtenfalls weitere Adsorptionsmittel wie z.B. Aktivkohle zufügt.

In der deutschen Patentanmeldung DE 38 21 151 wird vorgeschlagen, zur Geruchsminderung und farblichen Verbesserung Fermenterbrühen und/oder Enzymlösungen mit reduzierenden Zusätzen zu bearbeiten.

Ein ähnliches Verfahren wird in der deutschen Patentanmeldung P 39 30 284.9 beschrieben. Dort wird vorgeschlagen, daß man als selektive Adsorptionsmittel Zellen von Pilzen, Pflanzen und/oder Bakterien oder Zellwandstücke der o.g. Organismen einer Fermenterbrühe zusetzt. Auch die deutsche Patentanmeldung P 39 15 277.4 beschreibt ein ähnliches Verfahren. Vorgeschlagen wird über einen pH-Wert zwischen 5 und 11 der Enzymlösung eine saure wäßrige Lösung eines Aluminiumsalzes sowie gewünschtenfalls zusätzliche Fällungsmittel zuzusetzen, wasserlösliche Bestandteile abzutrennen und nach der Fällung ein Maskierungsmittel, wie eine Säure des Bors oder dergleichen zuzugeben.

Allen genannten Verfahren liegt die Idee zugrunde, die störenden Bestandteile an die Oberfläche eines Adsorptionsmittels zu binden oder mit der in-situ erzeugten Fällung eines Adsorptionsmittels mitzufällen, so daß eine Enzymlösung mit verbesserter Reinheit zurückbleibt. Zwar können nach diesen Verfahren sehr saubere Enzymlösungen hergestellt werden, doch sinkt die Enzymausbeute naturgegebenermaßen mit steigendem Einsatz, so daß in jedem Falle ein Kompromiß zwischen guter Qualität und guter Quantität eingegangen werden muß.

Es ist jedoch bereits bekannt, Proteine aus Fermenterlösungen durch Ausfällen nicht der störenden Beimengungen, sondern der Proteine selbst abzutrennen. Bei Einsatz der üblichen Fällungsmittel in den üblichen Konzentrationen werden dabei aber die Verunreinigungen mit gefällt, so daß die gewünschte Reinheit so nicht zu erzielen ist.

Der Erfindung liegt die überraschende Erkenntnis zugrunde, daß eine Konzentrationsfällung von Proteinen, insbesondere von Hydrolasen, dann möglich ist, wenn man durch eine vorhergehende Behandlung mit einem Adsorptionsmittel gewisse offenbar in Fermenterbrühen all gegenwärtige Stoffe, die die Konzentrationsfällung verhindern, abtrennt.

Gegenstand der Erfindung ist somit ein Verfahren zur Abtrennung von exocellulären Proteinen von Mikroorganismen aus einer filtrierten Fermenterbrühe, dadurch gekennzeichnet, daß man in einer ersten Stufe Stoffe, die die Proteinfällung behindern, mit Hilfe eines festen Adsorptionsmittel entfernt, die verbleibende Lösung auf einen Proteingehalt von etwa 30 bis 40 Gew.-% konzentriert und dann das Protein bei pH-Werten zwischen 6 und 10 ausfallen läßt und abtrennt, wobei gewünschtenfalls Fällungsmittel für Proteine zur Beschleunigung des Ausfallens zugesetzt werden können.

Das erfindungsgemäße Verfahren erlaubt es zahlreiche Proteine aufzureinigen, die durch Fermentation von Mikroorganismen erzeugt und als exocelluläre Proteine in den Fermenterbrühen vorliegen. So kann es insbesondere für die Enzymherstellung verwendet werden, so beispielsweise für die Herstellung von Proteasen, Amylasen, Cellulaser, Xylanasen, Pentosanasen oder Lipasen. Eine besondere Eignung zeigt das Verfahren bei der Herstellung von Proteasen, insbesondere von alkalischen Proteasen wie Serinproteasen.

Die für das erfindungsgemäße Verfahren geeigneten Fermenterlösungen stammen vorzugsweise aus der Kultivierung vor Mikroorganismen wie Bakterien oder Pilze, insbesondere aus der Kultivierung von Bacillus-Stämmen so beispielsweise von Stämmen von Bacillus subtilis, Bacillus licheniformis, Eacillus lentus oder dergleichen.

Gemäß der Erfindung werden die Fermenterbrühen zunächst mit einem Adsorptionsmittel behandelt. Dazu wird das Adsorptionsmittel in Mengen von typischerweise 0,5 bis 10 Gew.-%, bezogen auf Proteinlösung, zugegeben. Geeignete Adsoprtionsmittel sind beispielsweise silikatische Adsorptionsmittel wie Schichtsilikate, insbesondere Bentonite. Unter den Bentoniten eignen sich bevorzugt säureaktivierte Bentonite. So kann als besonders bevorzugte Bentonite ein säureaktivierter Bentonit mit einer Montmorillonit-Kennzahl von 70 und einer Feinheit von 93 % kleiner 100 µ eingesetzt werden.

Neben oder anstelle der Bentonite können als Fällungsmittel auch Aluminiumoxidhydrate oder allgemein gesprochen Aluminiumsalze eingesetzt werden, die bei dem um den Neutralpunkt liegenden pH-Bereich der Proteinlösungen abtrennbare Fällungen ergeben. Als Aluminiumsalze sind Aluminiumhydroxychloride bevorzugt. Unter dieser technischen Bezeichnung werden erfindungsgemäß Chlorhydroxyverbindungen des Aluminiums verstanden, z.E. (Al₂(OH)₅Cl) mit 2 bis 3 mol Kristallwasser. Bevorzugte Materialien sind hier technische Qualitäten, wie sie z.B. zur Wasserreinigung Verwendung finden. Weiter geeignet sind andere wasserlösliche Aluminiumsalze, die bei Anheben des pH-Werts in den Neutralbereich Fällungen von Aluminiumhydroxyden bilden. Die erfindungsgemäß eingesetzten Aluminiumsalze werden den Enzymlösungen in Form von sauren wäßrigen Lösungen beigegeben. Diese sauren Lösungen weisen einen pH-Wert um 3 bis 4 und eine Konzentration zwischen 10 und 50 Gew.-% auf. Weiter haben sich Lösugen mit einem Gehalt um 20 Gew.-% als günstig erwiesen. Darüber hinaus kann Aluminiumhydroxychlorid auch in Pulverform direkt in die Enzymlösungen eingerührt werden. Dies ist jedoch weniger bevorzugt.

Beim Fällungsvorgang sollen die Enzymlösungen einen pH-Wert zwischen 5 und 11, vorzugsweise zwischen 5 und 8, aufweisen, da außerhalb dieser pH-Grenzen zum einen die Enzymstabilität beeinträchtigt werden kann, zum anderen das Fällungsmittel teilweise wieder in Lösung gehen könnte. Der Fachmann hat daher darauf zu achten, daß bei Zugabe des Fällungsmittels der pH-Wert nicht unter 5 absinkt. Dies kann beispielsweise durch Zugabe von alkalischen Lösungen, z.B. von Natron- oder Kalilauge, verhindert weraen. Auch die Zugabe von Pufferlösungen ist denkbar, muß jedoch auf das Fällungsmittel abgestimmt werden.

Die einzusetzende Menge an Aluminiumsalz richtet sich nach dem zu erzielenden Reinigungsgrad. Für viele technische Anwendungen dürften Mengen von 1 bis 5 Gew.-% bevorzugt sein, jedoch werden bereits mit geringeren Einsatzmengen Reinigungseffekte erzielt, z.B. ab 0,5 Gew.-%. Höhere Einsatzmengen z.B. bis zu 10 Gew.-% sind möglich, dürften sich aber vielfach aus wirtschaftlichen Gründen verbieten.

Nach einer weiteren Ausführungsform der Erfindung kann auch mit unlöslichen Calciumsalzen gefällt werden. Bevorzugt ist die Erzeugung von Calciumphosphaten in-situ in den Proteinlösungen.

Dabei wird bevorzugt das Verhältnis des Calciumsalzes zum Verhältnis des Salzes der Phosphorsäure so gewählt, daß das Molverhältnis Calcium zu Phosphor zwischen 1,7 und 2,5 : 1 beträgt. Dabei hat sich herausgestellt, daß unter diesen Bedingungen Calciumphosphate mit überwiegend amorpher Struktur und großer Oberfläche entstehen, die für die hier zu fällenden farblichen Verunreinigungen günstige Adsorptionsmittel darstellen. Die Fällungsmittelmenge, bezogen auf Enzymlösung, beträgt üblicherweise 0,5 bis 20 Gew.-%.

Ein weiteres Adsorptionsmittel, das neben oder anstelle der genannten Adsorptionsmittel verwendet werden kann, ist Aktivkohle.

Nach einer weiteren Ausführungsform der Erfindung kann den Enzymlösungen vor oder nach der Fällung ein Maskierungsmittel zugesetzt werden. Bevorzugt ist der Zusatz des Maskierungsmittels vor der Fällung, da dabei geringere Verluste an Enzymaktivität erhalten werden.

Als Maskierungsmittel können zum einen Säuren des Bors und schwefelige Säuren sowie deren Alkalimetallsalze zugesetzt werden. Die zuzusetzenden Mengen betragen 0,5 bis 5 Gew.-%, vorzugsweise 1 bis 3 Gew.-%, bezogen auf Enzymlösungen, wobei sich größere Mengen in erster Linie aus wirtschaftlichen Gesichtspunkten verbieten. Geeignete Säuren des Bors sind die Borsäure, die Metaborsäure und/oder die Pentaborsäure. Geeignete Alkalimetallsalze sind daher insbesondere Natriumborat, Natriummetaborat, Borax oder Natriumpentaborat. Weiterhin geeignet ist das Natriumsulfit.

Als weitere Maskierungsmittel können zusammen mit den vorgenannten oder anstelle derselben Dicarbonsäuren und/oder Hydroxycarbonsäuren mit 3 bis 10 C-Atomen eingesetzt werden. Bevorzugt sind die Hydroxydicarbonsäuren, insbesondere Zitronensäure, Weinsäure und deren Isomere. Die Zusatzmenge beträgt 1 bis 5 Gew.-%. Auch hier verbieten sich höhere Zusatzmengen, in erster Linie aus wirtschaftlichen Gesichtspunkten und nicht wegen Nachlassen der technischen Effekte.

Am Ende der Behandlung mit dem Adsorptionsmittel wird die Enzymlösung aufkonzentriert. Dabei ist es bevorzugt, den Proteingehalt auf 40 bis 50 Gew.-% einzustellen. Der pH-Wert der Zubereitung sollte in der Nähe des Neutralpunkts liegen. Es ist bevorzugt, den pH zwischen 7,5 und 9 einzustellen. Dies gilt für Hydrolasen und insbesondere Proteasen ganz besonders.

Für die Herstellung der konzentrierten Enzymlösungen stehen dem Fachmann eine Reihe von Verfahren zur Verfügung. So kann dabei die Mikrofiltration und/oder Ultrafiltration eingesetzt werden, und es können dann die erhaltenen Enzymlösungen vorher oder anschließend durch Abdestillieren von Wasser unter vermindertem Druck, etwa in einem Dünnschichtverdampfer, auf noch höhere Konzentrationen gebracht werden. Nach einem ganz besonders bevorzugten Verfahrer wird zunächst durch Mikrofiltration und Ultrafiltration vorgereinigt, dann gefällt und schließlich durch Eindampfen konzentriert. Dabei werden die Mikrofiltations- und Ultrafiltrationsstufen insbesondere so ausgeführt, wie sie in der deutschen Patentanmeldung DE 37 30 858 beschrieben sind. Diese Patentanmeldung beschreibt ein Verfahren zur Abtrennung von biotechnologisch hergestellten Wertstoffen aus einer Fermenterbrühe durch Querstrom Mikro- und/oder Ultrafiltration, bei dem pro Stufe wenigstens zwei hintereinander geschaltete, mit porösen Membranen bestückte Module verwendet werden, das dadurch gekennzeichnet ist, daß an jedem Modul permeatseitig ein unterschiedlicher Überdruck gegenüber dem Umgebungsdruck angelegt wird. Zur Durchführung dieses Verfahrens bedient man sich vorzugsweise bei der Mikrofiltration einer Überströmungsgeschwindigkeit von mehr als 4 m/sec und setzt anorganische Materialien wie Aluminiumoxid, Siliciumcarbid oder Zirkoniumdioxid auf einem Träger als Membranwerkstoffe ein.

Die nach den bisher geschilderten Stufen des erfindungsgemäßen Verfahrens zubereiteten konzentrierten Proteinlösungen werden schließlich einem Fällungsschritt unterzogen. Vorzugsweise läßt an dabei das Protein ohne Zugabe weiterer Stoffe durch Abkühlen der Lösung in die Nähe des Gefrierpunkts und/oder durch Stehenlassen ausfällen. So ist es in vielen Fällen ausreichend, wenn die Lösung auf +5°C abgekühlt wird und über Nacht ohne weiteres Zutun gelagert wird.

Selbstverständlich ist es möglich, die konzentrierte Proteinlösung auch durch Zusatz von Proteinfällungsmitteln zu behandeln und dadurch die Proteine auszufällen. Diese Vorgehensweise ist jedoch nicht so sehr bevorzugt. Als Proteinfällungsmittel können beispielsweise lösliche Alkalimetallsalze in Mengen von 1 bis 5 Gew.-% oder wassermischbare organische Lösungsmittel in Mengen von 5 bis 20 Gew.-% oder wasserlösliche Polymere in Mengen von 0,1 bis 5 Gew.-% eingesetzt werden. An löslichen Alkalimetallsalzen sind Natriumchlorid, Natriumsulfat, Calciumchlorid und dergleichen bevorzugt und hier zu nennen. Geeignete wassermischbare Lösungsmittel sind mono- und difunktionelle Alkohole wie beispielsweise Ethylenglykol, Propylenglykol oder auch Methanol, Ethanol oder Aceton. Geeignete wasserlösliche Polymere sind Polyethylenglykol oder Polyacrylamid.

Die nach dem erfindungsgemäßen Verfahren erhaltenen gefällten Proteine können in üblicher Weise weiterverarbeitet werden. So können daraus wäßrige oder wäßrig-organische Proteinlösungen, beispielsweise Enzymkonzentratlösungen hergestellt werden. Andererseits ist auch die Konfektionierung zu Festprodukten möglich, in dem man die gefällten Proteine trocknet oder zusammen mit Zuschlagstoffen beispielsweise zu festen Enzymzubereitungen konfektioniert.

### Beispiele

### Beispiel 1

Durch Fermentation eines Bacillus licheniformis-Stammes, der eine exocelluläre Protease von Bacillus lentus erzeugt, wurden 200 1 einer Fermenterbrühe mit einer spezifischen Proteaseaktivität von 34850 HPE/g hergestellt und wie folgt weiterverarbeitet.

### Mikrofiltration

### Apparat:

| | |
|---|---|
| Typ | Rohrmodule |
| | Pilot-Anlage 2S151, Fa. TECHSEP, Frankreich |
| Filterfläche | 2 x 3,4 m (2 Module in Reihe) |
| Membranmaterial | Typ M14, Zirkonoxid auf Graphit |
| Trenngrenze | 0,14 µm |

### Betriebsbedingungen:

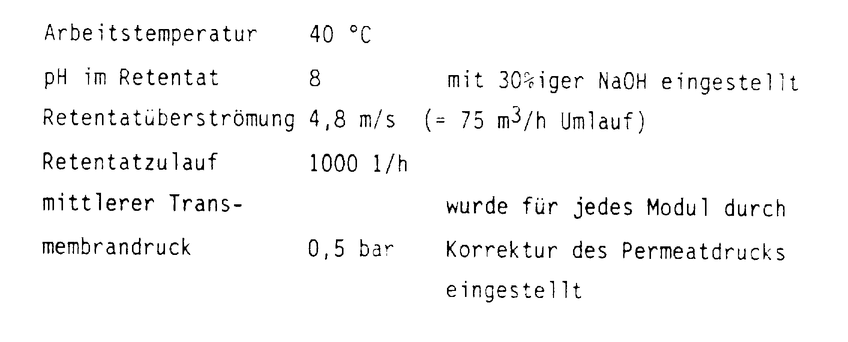

### Zusatz des Fällungsmittels:

Ein Aluminiumoxidchloridhydrat (Handelsname LOCRON (R)) wurde in Mengen von 50 g/l der Proteaselösung zugesetzt. Mit Natronlauge wurde ein pH-Wert von 8,0 eingestellt.

### Vorverdünnung:

Die 200 l Kulturlösung wurden zur Verringerung des Feststoffgehaltes und der Viskosität mit 140 l Salzlösung (NaCl Gewerbesalz, techn. 90%) verdünnt.

| | |
|---|---|
| Salzkonzentration | 10 g/l |
| Verdünnung τ | 0,59 |

### Diafiltration:

Unter Beibehalten des eingestellten Konzentrierungsfaktors τ = 0,59 wurden insgesamt 850 l Salzlösung (NaCl) zum Retentat nachdosiert.

| | |
|---|---|
| Salzkonzentration | 10 g/l |
| relatives Diafiltratvolumen | 4.25 l/l |
| Permeatstromdichte | 29 l/mh |

### Aufkonzentrierung:

Abschließend wurde das Retentat bis auf 170 l eingeengt.

| | |
|---|---|
| Konzentrierung τ | 1.2 |

### Ergebnis:

Insgesamt fielen aus Diafiltration und Aufkonzentrierung 1020 l enzymhaltiges Permeat an.
spezifische Proteaseaktivität 4520 HPE/g

### Ultrafiltration

### Apparat:

| | |
|---|---|
| Typ | Millipore Spiralmodul |
| Filterfläche | 5.6 m |
| Membranmaterial | Polysulfon |
| Trenngrenze | 10000 Dalton |

### Betriebsbedingungen:

| | |
|---|---|
| Arbeitstemperatur | 25°C |
| Rententatzulauf | 2500 l/h |
| mittl. Transmembrandruck | 1 bar |

### Aufkonzentrierung:

Die Aufkonzentrierung wird bis auf ca. 30 l vorgenommen. Dies entspricht einer Volumenreduktion um den Faktor 34, bzw. verglichen mit der Ausgangsmenge Kulturlösung

| | |
|---|---|
| Konzentrierung τ | 6.6 |

Die Permeatstromdichte sinkt während der Aufkonzentrierung von 20 l/mh auf 5 l/mh.

### Ergebnis:

30 l Konzentrat
spezifische Proteaseäktivität 112000 HPE/g.

### Thermische Aufkonzentrierung

### Apparat:

| | |
|---|---|
| Typ | Dünnschichtverdampfer |
| | Centritherm |
| | CT1B-2, Fa. α-Laval |
| Heizfläche | 0.09 M |
| Verdampferleistung | 50 kg/h (Wasser) |

### Betriebsbedingungen:

| | |
|---|---|
| Primäre Dampftemperatur | 80°C |
| sekundäre Dampftemperatur | 35°C |
| sekundärer Dampfdruck | 0.01 bar |

Die Leistung des Apparates liegt bei 12 kg/h (Zulauf).

### Aufkonzentrierung:

Die Aufkonzentrierung erfolgte über das übliche Maß hinaus. Auf die Ausgangsmenge wurde eine Volumenreduktion von über 40 erreicht.

| | |
|---|---|
| Konzentrierung in der Stufe τ | 6 |

### Ergebnis:

4.9 kg DSV-Konzentrat mit

| | |
|---|---|
| Trockensubstanz | 42.4 % |
| Proteaseaktivität | 755000 CPE/g. |

Das opak trübe Dünnschichtverdampferkonzentrat wurde über Nacht bei 5°C stehen gelassen. Es entstand ein weißer in einer Filternutsche abtrennbarer Niederschlag, der sich als ausgefälltes Enzymprotein erwies.

### HPE-Einheiten:

Bei der standardisierten HPE-Methode wird die Protease mit denaturiertem Casein bei 50°C, pH 8,5 über 15 Minuten inkubiert, überschüssiges Substrat durch Trichloressigsäure ausgefällt und die Extinktion des alkalisierten Überstandes bei 290 nm gemessen (lösliche aromatische Peptide). Nach Standardbedingungen entsprechen 0,5 Extinktionseinheiten 10 HPE. Die HPE-Methode ist mit der Anson-Methode vergleichbar.

### Enzymaktivitätseinheit CPE:

Das Testprinzip basiert auf dem proteolytischen Abbau von N,N-Dimethylcasein in Natrium-Sulfit-Lösung bei 50°C und anschließender Farbreaktion der freigesetzten Aminogruppen mit Trinitrobenzolsulfonsäure. Der Farbkomplex wird bei 425 nm photometrisch quantifiziert und gegen einen Proteasestandard ausgewertet.

## Patentansprüche

1. Verfahren zur Abtrennung von exocellulären Hydrolasen von Mikroorganismen aus einer filtrierten Fermenterbrühe, dadurch gekennzeichnet, daß man
- in einer ersten Stufe Stoffe, die die Proteinfällung behindern, mit Hilfe eines festen Adsorptionsmittel entfernt.
- die verbleibende Lösung auf einen Proteingehalt von etwa 30 bis 40 Gew.-% konzentriert und
- dann das Protein bei pH-Werten zwischen 6 und 10 ausfallen läßt und abtrennt.

2. Verfahren nach Anspruch 1, dadurch gekennzeichnet, daß man beim Ausfallen lassen Fällungsmittel für Proteine zur Beschleunigung des Ausfallens zusetzt.

3. Verfahren nach Anspruch 1, dadurch gekennzeichnet, daß als Hydrolasen, Proteasen, Amylasen, Cellulasen, Xylanasen oder Lipasen zugegen sind.

4. Verfahren nach einem der Ansprüche 1 bis 3, dadurch gekennzeichnet, daß als Proteasen alkalische Proteasen, insbesondere Serin-Proteasen zugegen sind.

5. Verfahren nach einem der Ansprüche 1 bis 4, dadurch gekennzeichnet, daß als Adsorptionsmittel Silikate, wie Bentonite, Calciumphosphat, Aktivkohle und/oder Aluminiumsalze eingesetzt werden.

6. Verfahren nach einem der Ansprüche 1 bis 5, dadurch gekennzeichnet, daß die Calciumphosphate in-situ aus löslichen Calciumsalzen und löslichen Phosphaten oder Phosphorsäuren hergestellt werden.

7. Verfahren nach den Ansprüchen 1 bis 5, dadurch gekennzeichnet, daß die Aluminiumoxidhydrate in-situ aus Aluminiumsulfathydraten oder Aluminiumchloridhydraten hergestellt werden.

8. Verfahren nach einem der Ansprüche 1 bis 7, dadurch gekennzeichet, daß der Proteingehalt auf 40 bis 50 Gew.-% eingestellt wird.

9. Verfahren nach einem der Ansprüche 1 bis 8, dadurch gekennzeichnet, daß ein pH-Wert von 7,5 bis 9 eingestellt wird.

10. Verfahren nach einem der Ansprüche 1 bis 9, dadurch gekennzeichnet, daß bei der Abtrennung von Hydrolasen enzymstabilisierende Mittel wie Borate und/oder mehrfunktionelle Carbonsäuren eingesetzt werden.

11. Verfahren nach einem der Ansprüche 1 bis 10, dadurch gekennzeichnet, daß die Aufkonzentrierung der Fermenterbrühe durch Mikrofiltration, Ultrafiltration und/oder Abdampfen des Wassers erfolgt.

12. Verfahren nach einem der Ansprüche 1 bis 11, dadurch gekennzeichnet, daß die Fermenterbrühe vor der Zugabe des Adsorptionsmittels durch Mikrofiltration und Ultrafiltration vorgereinigt wird, und daß nach der Zugabe des Adsorptionsmittels und dessen Abtrennung die erhaltenen Lösung bei vermindertem Druck auf die gewünschte Konzentration eingeengt wird.

13. Verfahren nach einem der Ansprüche 1 bis 12, dadurch gekennzeichnet, daß als Proteinfällungsmittel in der letzten Stufe lösliche Alkalimetallsalze in Mengen von 1 bis 5 Gew.-% , bezogen auf Proteinlösung, zugesetzt werden.

14. Verfahren nach einem der Ansprüche 1 bis 13, dadurch gekennzeichnet, daß in der letzten Verfahrensstufe als Proteinfällungsmittel organische Lösungsmittel in Mengen von 5 bis 20 Gew.-%, bezogen auf Protein, zugesetzt werden.

15. Verfahren nach einem der Ansprüche 1 bis 13, dadurch gekennzeichnet, daß in der letzten Verfahrensstufe als Proteinfällungsmittel wasserlösliche Polymere wie Polypropylenglykol in Mengen von 0,1 bis 5 Gew.-%, bezogen auf Protein, zugesetzt werden.

## Claims

1. A process for the separation of exocellular hydrolases of microorganisms from a filtered fermenter broth, characterized in that
- in a first step, substances which impede the precipitation of proteins are removed by means of a solid adsorbent,
- the remaining solution is concentrated to a protein content of around 30 to 40% by weight and
- the protein is subsequently precipitated at pH values of 6 to 10 and removed.

2. A process as claimed in claim 1, characterized in that precipitants for proteins are added during precipitation to accelerate the precipitation process.

3. A process as claimed in claim 1, characterized in that proteases, amylases, cellulases, xylanases or lipases are present as the hydrolases.

4. A process as claimed in any of claims 1 to 3, characterized in that alkaline proteases, especially serine proteases, are present as the proteases.

5. A process as claimed in any of claims 1 to 4, characterized in that silicates, such as bentonites, calcium phosphate, active carbon and/or aluminium salts, are used as the adsorbent.

6. A process as claimed in any of claims 1 to 5, characterized in that the calcium phosphates are produced in situ from soluble calcium salts and soluble phosphates or phosphorus acids.

7. A process as claimed in claims 1 to 5, characterized in that the aluminium oxide hydrates are produced in situ from aluminium sulfate hydrates or aluminium chloride hydrates.

8. A process as claimed in any of claims 1 to 7, characterized in that the protein content is adjusted to 40 to 50% by weight.

9. A process as claimed in any of claims 1 to 8, characterized in that a pH value of 7.5 to 9 is adjusted.

10. A process as claimed in any of claims 1 to 9, characterized in that enzyme-stabilizing agents, such as borates and/or polybasic carboxylic acids, are used in the separation of hydrolases.

11. A process as claimed in any of claims 1 to 10, characterized in that the fermenter broth is concentrated by microfiltration, ultrafiltration and/or evaporation of the water.

12. A process as claimed in any of claims 1 to 11, characterized in that the fermenter broth is prepurified by microfiltration and ultrafiltration before addition of the adsorbent and in that, after addition of the adsorbent and its separation, the solution obtained is concentrated under reduced pressure to the required concentration.

13. A process as claimed in any of claims 1 to 12, characterized in that soluble alkali metal salts are added as protein precipitants in the final stage in quantities of 1 to 5% by weight, based on protein solution.

14. A process as claimed in any of claims 1 to 13, characterized in that organic solvents are added as protein precipitants in the final stage of the process in quantities of 5 to 20% by weight, based on protein.

15. A process as claimed in any of claims 1 to 13, characterized in that water-soluble polymers, such as polypropylene glycol, are added as protein precipitants in the final stage of the process in quantities of 0.1 to 5% by weight, based on protein.

## Revendications

1. Procédé de séparation d'hydrolases exocellulaires de microorganismes à partir d'un bouillon de fermenteur filtré, caractérisé en ce que l'on
- élimine, dans une première étape, les matières qui empêchent la précipitation des protéines, au moyen d'un adsorbant solide,
- concentre la solution restante jusqu'à une concentration en protéine d'environ 30 à 40 % en poids et que l'on
- précipite ensuite et sépare la protéine à un pH compris entre 6 et 10

2. Procédé selon la revendication 1, caractérisé en ce que l'on additionne des précipitants pour les protéines lors de la précipitation, afin d'accélérer la précipitation.

3. Procédé selon la revendication 1, caractérisé en ce que sont présentes comme hydrolases, des protéases, des amylases, des cellulases, des xylanases ou des lipases.

4. Procédé selon une des revendications 1 à 3, caractérisé en ce que sont présentes comme protéases, des protéases alcalines, en particulier des protéases de sérine.

5. Procédé selon une des revendications 1 à 4, caractérisé en ce que l'on met en oeuvre comme adsorbants, des silicates tels la bentonite, du phosphate de calcium, du charbon activé et/ou des sels d'aluminium.

6. Procédé selon une des revendications 1 à 5, caractérisé en ce que les phosphates de calcium sont synthétisés in-situ à partir de sels de calcium solubles et de phosphates solubles ou d'acides phosphoriques.

7. Procédé selon les revendications 1 à 5, caractérisé en ce que les hydrates d'oxyde d'aluminium sont produits in-situ à partir d'hydrates de sulfate d'aluminium ou d'hydrates de chlorure d'aluminium.

8. Procédé selon une des revendications 1 à 7, caractérisé en ce que la concentration en protéines est ajustée à 40 à 50 % en poids.

9. Procédé selon une des revendications 1 à 8, caractérisé en ce qu'un pH de 7,5 à 9 est ajusté.

10. Procédé selon une des revendications 1 à 9, caractérisé en ce que l'on met en oeuvre, lors de la séparation des hydrolases, des agents stabilisant les enzymes tels des borates et/ou des acides carboxyliques polyfonctionnels.

11. Procédé selon une des revendications 1 à 10, caractérisé en ce que la concentration du bouillon de fermenteur est opérée par microfiltration, ultrafiltration et/ou évaporation de l'eau.

12. Procédé selon une des revendications 1 à 11, caractérisé en ce que le bouillon de fermenteur fait l'objet d'une épuration préalable par microfiltration et ultrafiltration avant l'adjonction de l'adsorbant, et en ce qu'après l'adjonction de l'adsorbant et sa séparation, la solution obtenue est concentrée à la concentration souhaitée sous pression réduite.

13. Procédé selon une des revendications 1 à 12, caractérisé en ce que l'on additionne comme précipitant de protéines au cours de la dernière étape, des sels solubles de métaux alcalins, en proportions de 1 à 5 % en poids, par rapport à la solution de protéines.

14. Procédé selon une des revendications 1 à 13, caractérisé en ce que l'on additionne comme précipitant de protéines, au cours de la dernière étape du procédé, des solvants organiques en proportions de 5 à 20 % en poids par rapport à la protéine.

15. Procédé selon une des revendications 1 à 13, caractérisé en ce que l'on additionne comme précipitant de protéines, au cours de la dernière étape du procédé, des polymères solubles dans l'eau tels que du polypropylèneglycol, en quantités de 0,1 à 5 % en poids par rapport à la protéine.
